# FASCICULE DE BREVET EUROPEEN

(11) **EP 2 243 444 B1**
(45) Date de publication et mention de la délivrance du brevet: **02.01.2013**
(21) Numéro de dépôt: 10160400.7
(22) Date de dépôt: 20.04.2010
(51) Int. Cl.: A61F 2/40

(54) **Dispositif de fixation à la glène d'un composant articulaire glénoïdien pour prothèse d'épaule, ainsi que prothèse d'épaule correspondante**
Vorrichtung zum Befestigen der Gelenkschale einer Schulterprothese sowie entsprechende Schulterprothese
Device for fixation of a joint cup for a shoulder prosthesis and corresponding shoulder prosthesis

(30) Priorité: 22.04.2009 FR 0952635
(43) Date de publication de la demande: 27.10.2010
(73) Titulaire: Tornier, 38330 Montbonnot-Saint-Martin (FR)
(72) Inventeur: Brunnarius, Yann, 26300, CHATUZANGE LE GOUBET (FR); Deransart, Pierric, 38000, GRENOBLE (FR)
(74) Mandataire: Grand, Guillaume

(56) Documents cités:
- EP-A- 1 402 853
- EP-A- 1 639 967
- US-A- 5 593 448
- US-A- 5 800 551
- US-A1- 2003 055 507
- US-A1- 2007 260 321

## Description

La présente invention concerne un dispositif de fixation à la glène d'un composant articulaire glénoïdien pour prothèse d'épaule. Elle concerne également une prothèse d'épaule comportant un tel dispositif de fixation.

Une des causes d'échec ou de complication d'une arthroplastie de l'épaule est liée à une mauvaise fixation du corps prothétique glénoïdien à la glène. En effet, lorsque la glène d'un patient opéré est usée et/ou présente une constitution osseuse altérée, le chirurgien a des difficultés, d'une part, à positionner correctement le corps prothétique glénoïdien par rapport à la glène, en vue d'imposer à la face articulaire de ce composant une rétroversion identique à ou, à tout le moins, aussi proche que possible de la rétroversion anatomique de la glène originelle du patient, et, d'autre part, à fixer fermement le corps prothétique à la glène, en vue de garantir une résistance de fixation suffisante. Une fixation mal positionnée et/ou insuffisamment résistante conduit à l'usure et/ou au descellement du corps prothétique glénoïdien.

Dans ces conditions, US-A-5 800 551, US-A-5 593 448 et US-A-2003/0055507 ont proposé de munir la face du composant glénoïdien, opposée à sa face articulaire, de plusieurs pions saillants, conçus pour être enfoncés dans la matière osseuse spongieuse remplissant la fosse de la glène du patient opéré, le cas échéant jusqu'à ce que l'un ou certains de ces pions soient appuyés par leur extrémité libre contre le fond de la voûte osseuse corticale de la glène. En pratique, le positionnement de ces pions vis-à-vis de la glène reste délicat en présence d'une glène usée. En outre, dans la mesure où la transmission d'efforts est nécessairement limitée entre l'extrémité libre des pions et la glène, il est nécessaire de prévoir que la périphérie du composant glénoïdien soit placée en appui contre la bordure d'extrémité, tournée vers l'humérus, de la voûte de la glène, ce qui, à la longue, fragilise cette bordure d'extrémité par apparition de liserés et conduit au descellement du composant glénoïdien.

EP-A-1 639 967 a, quant à lui, envisagé de munir la face du composant glénoïdien, opposée à sa face articulaire, d'une ancre saillante massive, en forme globale de tronc de cône, pour s'appuyer latéralement sur la paroi de la voûte de la glène, en occupant tout le volume interne de la fosse de cette glène. Cette solution interdit toute recolonisation osseuse de la fosse et conduit rapidement à la nécrose de la glène, d'autant que, comme précédemment, la périphérie du composant glénoïdien est prévue appuyée contre la bordure d'extrémité de la voûte de la glène.

Pour sa part, US-A-2007/0260321, sur lequel est basé le préambule de la revendication 1, divulgue un corps de support d'un composant glénoïdien, ce corps étant dimensionné pour s'étendre à l'intérieur de la voûte osseuse corticale d'une glène. Ce corps est pourvu extérieurement de quatre ardillons (désignés par le terme anglais « barbs » dans ce document) : d'après les explications fournies dans ce document, la conformation extérieure tronconique du corps est prévue pour permettre d'emmancher à force ce corps à l'intérieur de la voûte de la glène (comme indiqué par l'expression « press fit » du texte de ce document), tandis que les ardillons précités permettent de verrouiller en place le corps, par un effet de retenue mécanique. Par conséquent, on comprend que ces ardillons sont incapables de transmettre de fortes contraintes mécaniques du corps à la paroi périphérique de la voûte glénoïdienne puisque, au contraire, dans US-A-2007/0260321, de telles contraintes sont transmises directement du corps à la paroi périphérique de la voûte. De plus, eu égard à sa forme extérieure tronconique, le corps de support envisagé dans ce document s'appuie latéralement sur la paroi périphérique de la voûte glénoïdienne, en occupant la totalité du volume interne de la fausse de cette glène : par conséquent, ce dispositif interdit toute recolonisation osseuse de la fosse et conduit rapidement à la nécrose de la glène.

Le but de la présente invention est de proposer un dispositif de fixation d'un composant prothétique glénoïdien, qui à la fois soit facile à positionner sur la glène, même lorsque cette dernière est usée, fournit une liaison résistante avec la glène et respecte la constitution osseuse de la glène.

A cet effet, l'invention a pour objet un dispositif de fixation à la glène d'un composant articulaire glénoïdien pour prothèse d'épaule, tel que défini à la revendication 1.

L'idée à la base de l'invention est de chercher à mettre à profit la forme géométrique et la tenue mécanique de la voûte de la glène du patient opéré, cette voûte étant constituée d'os cortical, sans nuire à l'environnement biologique de cette voûte, notamment en garantissant son irrigation sanguine. Pour ce faire, l'invention prévoit d'utiliser des bras pour s'appuyer transversalement contre la face interne de la paroi périphérique de la voûte de la glène : ces bras permettent une fixation par ancrage particulièrement résistante, dans le sens où l'étendue du contact entre ces bras et la paroi périphérique de la voûte permet une transmission d'efforts importante, sans endommager pour autant la glène puisque c'est la matière osseuse corticale de cette dernière qui est sollicitée. Les bras d'appui sont supportés mécaniquement par le corps central du dispositif selon l'invention. Ce corps permet de centrer globalement le dispositif dans la fosse de la glène, même si la glène du patient opéré est usée, en plaçant ce corps de manière qu'il s'étende sensiblement suivant l'axe géométrique central de révolution associé à la fosse. De surcroît, comme les bras délimitent entre eux des espaces libres à la surface du corps, l'irrigation biologique de la fosse est ainsi permise jusqu'au fond de la voûte, ce qui favorise la colonisation osseuse des espaces libres précités et ainsi une bonne fixation secondaire du dispositif selon l'invention.

D'autres caractéristiques avantageuses du dispositif de fixation conforme à l'invention, prises isolément ou suivant toutes les combinaisons techniquement possibles, sont spécifiées aux revendications dépendantes 2 à 9

L'invention a également pour objet une prothèse d'épaule, telle que définie à la revendication 10.

Ainsi, la prothèse d'épaule conforme à l'invention peut donc avoir sa face articulaire concave pour s'articuler sur une tête humérale convexe, la prothèse étant soit une prothèse d'hémi-arthroplastie si la tête humérale précitée est naturelle, soit une prothèse totale dite standard, si la tête humérale est prothétique. La prothèse d'épaule selon l'invention peut également être une prothèse totale dite inversée, dont la face articulaire du composant glénoïdien est convexe pour s'articuler avec un insert huméral prothétique concave.

On propose également ici une méthode chirurgicale de fixation à la glène d'un composant articulaire glénoïdien, dans laquelle :
- on accède à l'intérieur de la voûte osseuse corticale de la glène,
- on introduit à l'intérieur de cette voûte un corps de support du composant glénoïdien et on appuie contre la face interne de la paroi périphérique de cette voûte au moins trois bras, dont le corps est pourvu solidairement, de manière à délimiter, entre ces bras, des passages libres autour du corps, qui courent sur la face extérieure de ce corps ; et
- on rapporte le composant glénoïdien sur le corps.

L'invention sera mieux comprise à la lecture de la description qui va suivre, donnée uniquement à titre d'exemple et faite en se référant aux dessins sur lesquels :
- la figure 1 est une vue en perspective d'une prothèse d'épaule conforme à l'invention, associée à l'omoplate d'un patient opéré et montrée avant son implantation sur cette omoplate ;
- la figure 2 est une vue en perspective, sous un angle de vue différent, de la prothèse de la figure 1 ;
- les figures 3 et 4 sont des coupes selon le plan III de la figure 1, montrant respectivement le dispositif de fixation appartenant à la prothèse, en cours d'implantation, et la prothèse après son implantation sur l'omoplate ;
- la figure 5 est une vue en élévation d'un autre mode de réalisation d'une prothèse d'épaule conforme à l'invention ;
- la figure 6 est une vue éclatée en perspective d'un autre mode de réalisation du dispositif de fixation conforme à l'invention ; et
- la figure 7 est une coupe longitudinale du dispositif de la figure 6, à l'état assemblé.

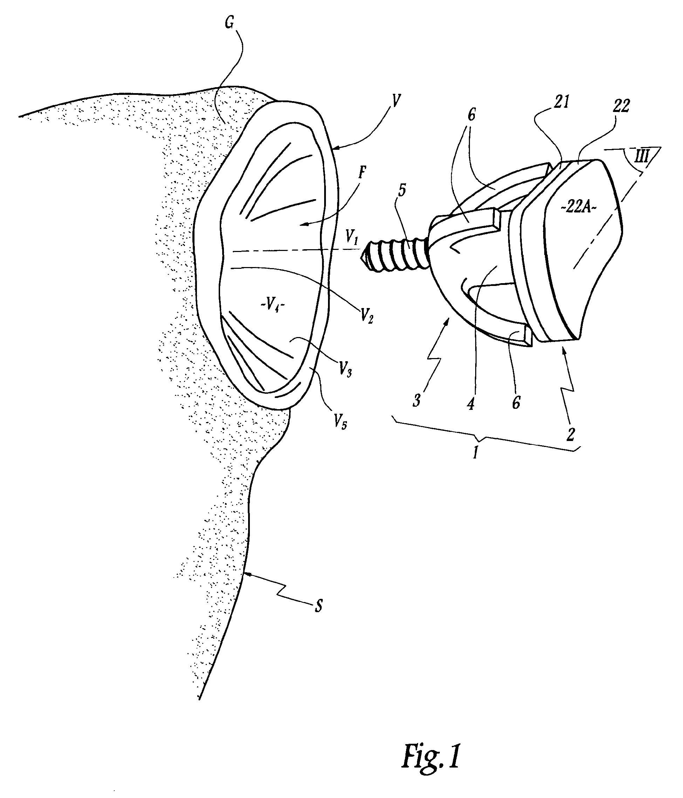

Sur les figures 1 à 4 est représentée une prothèse d'épaule 1 dite standard ou non inversée, destinée à être implantée sur l'omoplate S d'un être humain. Cette prothèse 1 comporte un composant glénoïdien 2 qui, après sa fixation à la glène G de l'omoplate S par un dispositif 3 détaillé ci-après, est adapté pour s'articuler avec une tête, éventuellement prothésée, de l'humérus, non représenté, du patient opéré, en vue de reproduire un comportement articulaire aussi proche que possible du comportement d'origine de l'épaule du patient.

Dans l'exemple de réalisation considéré sur les figures 1 à 4, le composant glénoïdien 2 comporte une embase 21, réalisée notamment en métal, et un patin 22, réalisé notamment en polyéthylène. D'autres couples de matériaux sont connus dans la technique, avec deux matériaux identiques ou différents. Ce patin 22 est solidarisé fixement à l'embase 21 par tout moyen approprié tandis que, sur son côté opposé à l'embase, le patin délimite une face concave 22A conformée pour s'articuler avec une face articulaire complémentaire délimitée par la tête, prothétique ou osseuse, de l'humérus du patient opéré.

Le dispositif 3 est conçu pour fixer par ancrage le composant glénoïdien 2 à la voûte V de la glène G. Cette voûte V est une structure osseuse anatomique, constituée de matière osseuse corticale, c'est-à-dire de matière osseuse dure et mécaniquement résistante, et présentant une géométrie interne évasée vers l'humérus, en étant globalement centrée sur un axe géométrique de révolution V₁. La voûte V est ainsi constituée d'un fond fermé V₂ qui se prolonge par une paroi périphérique V₃ dont la face interne V₄ est sensiblement centrée sur l'axe V₁, en s'évasant progressivement depuis le fond V₂ jusqu'à la bordure d'extrémité libre V₅ de la voûte V.

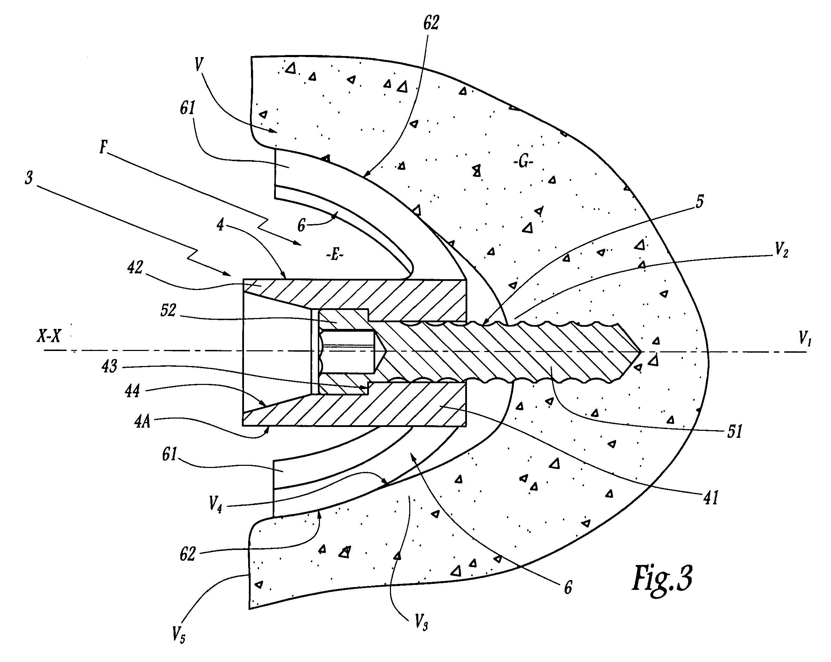

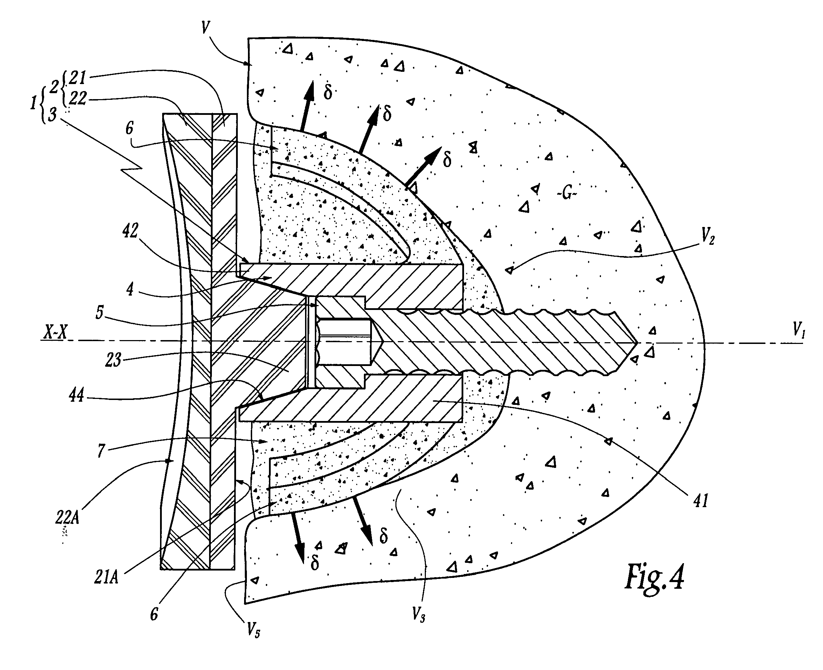

Le dispositif de fixation 3 comprend un corps allongé tubulaire 4, centré sur un axe X-X et dont la face extérieure 4A est ici cylindrique à base circulaire, centrée sur l'axe X-X. Ce corps 4 est dimensionné pour être reçu dans le volume interne de la voûte V, c'est-à-dire la fosse F de la glène G. Plus précisément, en alignant les axes X-X et V₁, le corps 4 peut être placé dans la fosse F, en s'étendant en longueur de manière que l'une de ses extrémités longitudinales, référencée 41, soit située à proximité du fond V₂ de la voûte V tandis que son extrémité longitudinale opposée 42 est située sensiblement au même niveau, selon l'axe V₁, de la bordure d'extrémité libre V₅ de la voûte, comme montré sur les figures 3 et 4. Lorsque le corps 4 est ainsi reçu dans la fosse F, sa face extérieure 4A et la face interne V₄ de la voûte V délimite entre elles un espace libre E, qui s'étend de manière périphérique tout autour du corps 4, comme bien visible sur la figure 3.

L'extrémité 41 du corps 4 est adaptée pour être relié fixement au fond V₂ de la voûte V par une vis 5. Pour ce faire, dans le mode de réalisation considéré ici, l'extrémité de corps 41 est conçue pour recevoir la vis 5, de manière centrée sur l'axe X-X, avec la tige 51 de cette vis qui s'étend pour partie à travers l'extrémité de corps 41 et dont le reste s'étend axialement en saillie du chant de cette extrémité de corps 41, tandis que la tête 52 de la vis 5 est disposée à l'intérieur du corps 4, en étant appuyé axialement contre un épaulement interne complémentaire 43 du corps 4, comme montré sur la figure 3.

L'extrémité 42 du corps 4 est, quant à elle, adaptée pour être liée fixement au composant glénoïdien 2. Dans le mode de réalisation considéré sur les figures, l'extrémité de corps 42 délimite à cet effet une surface intérieure tronconique 44, centrée sur l'axe X-X et convergente vers l'extrémité de corps 41. Comme montré sur la figure 4, cette surface 44 est ainsi conformée pour recevoir de manière complémentaire un pion tronconique 23 s'étendant en saillie depuis la face 21 A de l'embase 21, opposée au patin 22. En emboîtant le pion 23 dans la surface 44, l'embase 21 et donc tout le composant 2 se trouvent bloqués vis-à-vis du corps 4, comme représenté sur les figures 1, 2 et 4.

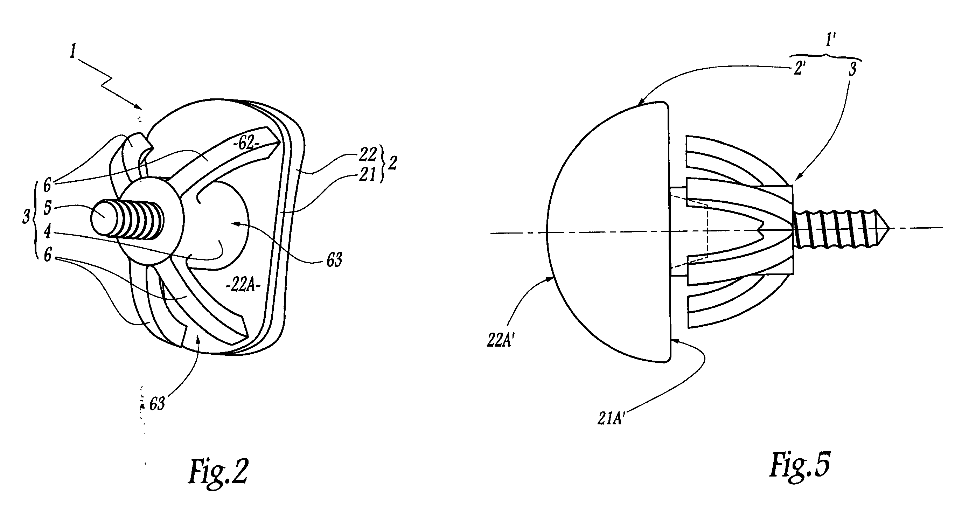

De plus, le corps 4 est pourvu de quatre bras 6, réalisés ici de manière monobloc avec le corps. Chacun des bras 6 est saillant depuis la face extérieure 4A du corps 4, au niveau de l'extrémité de corps 41, et s'étend en longueur le long du corps 4, en s'éloignant, radialement à l'axe X-X, de la face extérieure 4A de manière progressive, jusqu'à son extrémité libre référencée 61. Chaque bras 6 présente ainsi un profil longitudinal globalement incurvé, bombé à l'opposé du corps 4, comme bien visible sur les figures 1 et 2. Chaque bras 6 présente ainsi, sur son côté opposé au corps 4, une surface convexe 62 qui, comme expliqué en détail plus loin, est conformée pour s'appuyer de manière sensiblement complémentaire contre la face interne V₄ de la paroi V₃ de la voûte V lorsque le corps 4 est logé dans la fosse F comme sur les figures 3 et 4. Dans l'exemple de réalisation considéré ici, chaque bras 6 présente une section transversale à profil extérieur sensiblement carré, de sorte que la surface 62 s'apparente à une portion de cylindre centrée sur un axe sensiblement orthoradial à l'axe X-X.

Les quatre bras 6 sont répartis autour du corps 4, ici de manière irrégulière, de manière à délimiter entre deux bras successifs, autour de ce corps, un passage libre 63 qui court sur toute la longueur de la face extérieure 4A du corps 4, en reliant ainsi les chants des extrémités 41 et 42.

Un exemple d'implantation de la prothèse d'épaule 1 est le suivant.

Dans un premier temps opératoire, un chirurgien dégage les matières molles entourant la glène G de l'omoplate S d'un patient, afin d'avoir accès à la voûte V et à la fosse F, comme représenté sur la figure 1. Le cas échéant, le chirurgien retire tout ou partie de la matière osseuse spongieuse résiduelle située dans la fosse F, de manière à bien dégager la face interne V₄ de la paroi périphérique V₃ de la voûte. En variante, cette matière osseuse n'est que peu, voire pas retirée, en étant alors compactée de manière à délimiter, à sa surface libre, c'est-à-dire sa surface tournée à l'opposé du fond V₂ de la voûte, une empreinte de réception du dispositif 3 : le contour de cette empreinte correspond sensiblement à la trace géométrique du dispositif 3 projeté dans un plan perpendiculaire à l'axe X-X.

Dans un deuxième temps opératoire, le chirurgien manipule le dispositif 3 :
alors que le composant glénoïdien 2 est absent, il introduit le corps 4 dans la fosse F, en alignant sensiblement les axes X-X et V₁, jusqu'à placer ce corps comme sur la figure 3. Les bras 6 se trouvent ainsi également logés dans la fosse F, avec leur surface 62 positionnée en appui contre la face V₄ de la paroi V₃ de la voûte V. La géométrie convexe des surfaces d'appui 62 est telle que ces surfaces épousent cette face V₄ de la voûte V, en s'étendant entre la bordure d'extrémité V₅ et la périphérie du fond V₂ de la voûte. A titre optionnel avantageux, la matière reliant chacun des bras 6 et le corps 4 peut présenter une certaine capacité de déformation souple, de manière à favoriser l'adaptation spatiale de ces bras vis-à-vis de la voûte V.

Le cas échéant, lors de cette mise en place du corps 4 et des bras 6 à l'intérieur de la fosse F, le chirurgien peut ajuster la position angulaire du corps 4 autour de l'axe X-X, pour maximiser l'étendue du contact entre les différentes surfaces d'appui 62 et la paroi périphérique V₃ de la voûte. On comprend ainsi l'intérêt que les bras 6 soit répartis de manière irrégulière autour du corps 4, étant remarqué que, généralement, la section transversale de la voûte des glènes humaines ne présente pas un profil intérieur rigoureusement circulaire.

Simultanément ou postérieurement à la mise en place du corps 4 et des bras 6 dans la fosse F, la vis 5 est introduite à l'intérieur du corps 4, depuis son extrémité 42, de manière à faire s'étendre partiellement sa tige 51 en saillie axiale de l'extrémité de tige 41 et à visser cette partie de tige saillante dans la matière osseuse constituant le fond V₂ de la voûte V. Ce vissage intraosseux, combiné au fait que la tête 52 de la vis est appuyée contre l'épaulement 43 du corps 4, permet, d'une part, de fournir une fixation primaire du dispositif 3 vis-à-vis de la glène G et, d'autre part, d'appuyer fermement les surfaces 62 des bras 6 contre la paroi V₃ de la voûte V. Le dispositif 3 se trouve ainsi fermement immobilisé par rapport à la glène G, tout en étant positionné correctement, c'est-à-dire de manière centrée sur l'axe V₁ de la voûte V, et ce même si la glène est localement usée. Le dispositif 3 est alors dans la configuration représentée sur la figure 3.

Dans un troisième temps opératoire, optionnel mais avantageux, le chirurgien introduit un greffon osseux spongieux 7 à l'intérieur de la fosse F. En pratique, ce greffon présente une consistance suffisamment malléable pour être rapporté autour du corps 4, notamment dans les passages libres 63 entre les bras 6 et peut ainsi atteindre, le cas échéant, la région du fond V₂ de la voûte V. L'espace libre périphérique E entre le corps 4 et la paroi V₃ de la voûte se trouve ainsi comblé par ce greffon 7, comme représenté sur la figure 4. La présence du greffon 7 améliore remarquablement la fixation secondaire du dispositif 3 à la glène G.

Dans un quatrième temps opératoire, le chirurgien rapporte le composant glénoïdien 2 sur le dispositif 3 et le lie fixement au corps 4, par emboîtement tronconique du pion 23 dans l'extrémité de tige 42. La prothèse d'épaule 1 est alors dans la configuration représentée sur la figure 4.

En service, la prothèse d'épaule 1 est sollicitée mécaniquement par la tête de l'humérus associée à l'omoplate S. Il en résulte que le composant 2 transmet des contraintes mécaniques au corps 4, ces contraintes étant encaissées par la voûte V, essentiellement par sa paroi périphérique V₃ via les surfaces d'appui 62, comme indiqué par les flèches δ sur la figure 4. Ces contraintes mécaniques se trouvent ainsi réparties sur une étendue de contact importante et sont supportées par une partie structurelle corticale de la glène G, ce qui rend la fixation du composant 2 particulièrement résistante. Autrement dit, le risque de descellement de ce composant est limité, voire quasiment nul.

En outre, dans ces conditions, le corps 4 et/ou la face 22A du composant 2 sont avantageusement dimensionnés pour maintenir la région périphérique de la face 21 A en contact peu pressant avec la bordure d'extrémité libre V₅ de la voûte V, voire à distance de cette bordure comme représenté sur la figure 4. Un tel agencement n'est pas préjudiciable à la tenue mécanique de la prothèse 1, puisque les bras 6 sont suffisants pour ancrer fermement la prothèse vis-à-vis de la glène G. En revanche, en prévoyant que la face 21 A du composant 2 ne s'appuie que peu, voire pas contre la bordure d'extrémité V₅, on évite la fragilisation de cette bordure d'extrémité par création de liserés, qui, à long terme, altèrent la tenue mécanique de la voûte V et conduisent généralement au descellement des prothèses.

Par ailleurs, le composant 2 à face articulaire 22A concave peut être remplacé par un composant 2', dont la face articulaire 22A' est convexe, tandis que sa face opposée 21 A' est fonctionnellement analogue à la face 21 A du composant 2, dans le sens où elle permet de lier fixement ce composant 2' au dispositif de fixation 3 par emboîtement tronconique, comme représenté sur la figure 5. La prothèse d'épaule 1', comprenant le composant 2' et le dispositif 3, correspond ainsi à une prothèse d'épaule inversée, destinée à coopérer avec un composant prothétique huméral à face articulaire concave, complémentaire de la face 22A'.

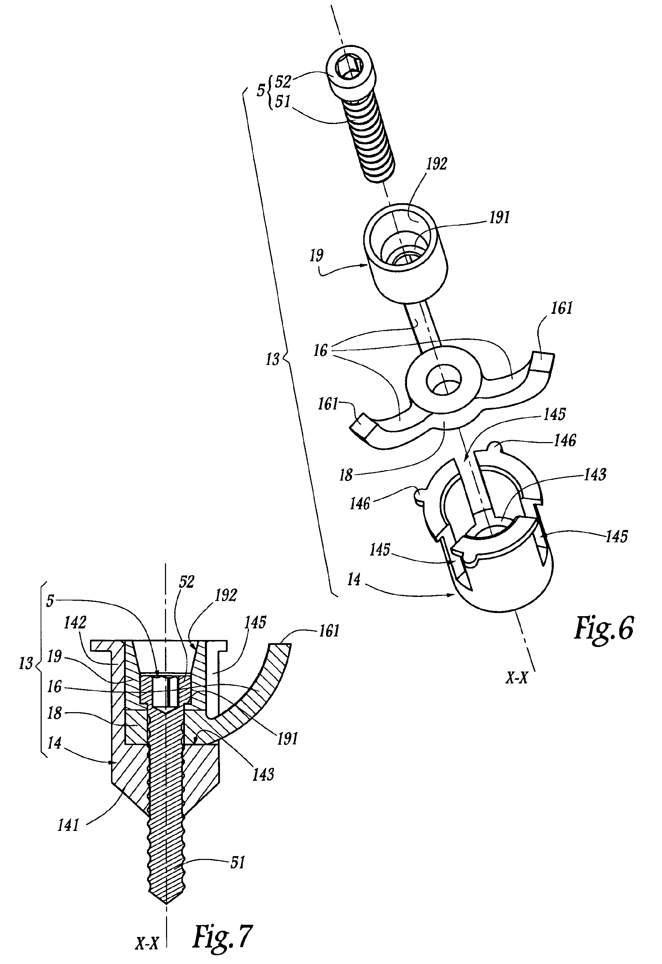

Sur les figures 6 et 7 est représenté un dispositif 13 correspondant à un mode de réalisation alternatif du dispositif de fixation 3 décrit jusqu'ici. Le dispositif 13 se distingue du dispositif 3 essentiellement par le fait que ses bras 16 ne sont pas réalisés d'une seule pièce avec son corps tubulaire 14. Plus précisément, les trois bras 16 sont portés par une même bague de support 18, distincte du corps 14, en étant ici venus de matière avec cette bague. La bague 18 relie ainsi les unes aux autres les extrémités des bras 16, opposées à leur extrémité libre respective 161. De plus, cette bague 18 est dimensionnée pour être rapportée à l'intérieur du corps 14, en étant centrée sur l'axe X-X de ce corps, comme représenté sur la figure 7. Pour ce faire, la bague 18 est introduite à l'intérieur du corps 14, depuis l'extrémité axiale 142 de ce corps, opposée à son extrémité 141 destinée, en service, à être tournée vers le fond V₂ de la voûte V.

Pour permettre à la bague 18 d'être engagée à l'intérieur du corps 14 jusqu'à prendre appui axialement contre un épaulement interne 143 de ce corps, ce dernier délimite des fentes traversantes rectilignes 145, parallèles à l'axe X-X et conformées chacune pour recevoir l'un des bras 16. Chaque fente 145 s'étend ainsi depuis l'épaulement 143 jusqu'à l'extrémité 142 du corps 14, où elle débouche librement sur l'extérieur.

En plus de la vis 5 décrite plus haut, le dispositif 13 comporte une douille 19 adaptée pour être rapportée co-axialement à l'intérieur du corps 14, plus précisément à l'intérieur de l'extrémité 142 de ce dernier, avec interposition axiale de la bague 18 entre cette douille 19 et l'épaulement 143, comme représenté sur la figure 7. L'alésage de la douille 19 comprend, dans sa partie tournée vers la bague 18 lorsque la douille est assemblée au corps 14, un épaulement 191 contre lequel la tête 52 de la vis 5 s'appuie axialement lorsque cette vis est introduite par sa tige 51 dans la douille, tandis que, dans sa partie opposée, l'alésage précitée forme une surface tronconique 192 qui, lorsque la douille est assemblée au corps, est centrée sur l'axe X-X et convergente vers l'extrémité 141 de ce corps. En service, l'épaulement 191 et la surface tronconique 192 sont fonctionnellement analogues à l'épaulement 43 et à la surface tronconique 44 du dispositif de fixation 3, vis-à-vis respectivement de la vis 5 et du composant glénoïdien 2 ou 2'.

Un exemple d'utilisation du dispositif 13 pour fixer le composant 2 ou 2' à la glène G est le suivant. Après avoir accédé à la voûte V de la glène G, le chirurgien introduit le corps 14 dans la fosse F, en alignant sensiblement les axes X-X et V₁, le cas échéant jusqu'à appuyer l'extrémité 141 de ce corps contre le fond V₂ de la voûte. Puis, le chirurgien rapporte successivement à l'intérieur du corps 14, en les passant à travers l'extrémité de corps 142, la bague 18, la douille 19 et la vis 5. La progression de la bague 18 jusqu'à l'épaulement 143 nécessite d'engager chacun des bras 16 dans l'une des fentes 145 : pour faciliter le positionnement angulaire relatif entre le corps 14 et la bague 18, l'extrémité 142 de ce corps est avantageusement munie de saillies de repérage visuel 146.

Le vissage de la tige 51 de la vis 5 dans la matière osseuse constituant le fond V₂ de la voûte V fixe l'assemblage des différents composants du dispositif 13, tout en fournissant la fixation primaire de ce dispositif vis-à-vis de la glène G et en appuyant contre la paroi V₃ de la voûte V les surfaces 162 des bras 16, opposées au corps 14.

Bien entendu, en variante, la bague 18 et la douille 19 peuvent être rapportées à l'intérieur du corps 14 avant que ce dernier ne soit introduit dans la fosse F.

En rapportant ensuite le composant glénoïdien 2 ou 2' sur le dispositif 13, par emboîtement tronconique dans la douille 19, on termine l'implantation d'une prothèse d'épaule comprenant le composant 2 ou 2' et le dispositif 13. Bien entendu, le cas échéant, les premier et troisième temps opératoires évoqués plus haut sont mis en oeuvre dans le cadre de l'implantation de la prothèse d'épaule précitée.

Par ailleurs, suivant une autre variante, non représentée, des dispositifs de fixation 3 et 13, les bras 6 ou 16 sont reliés respectivement au corps 4 ou à la bague 18 par des moyens mécaniques articulés, adaptés pour autoriser le basculement de chaque bras par rapport au corps autour d'un axe d'articulation orthoradial à l'axe X-X. Le basculement des bras 6 ou 16 autour des axes précités est alors avantageusement commandé par la vis 5 lorsque cette dernière est introduite à travers l'extrémité 41 ou 141 du corps 4 ou 14, pour être vissée dans le fond V₂ de la voûte V. L'intensité de l'ancrage des bras 6 ou 16 vis-à-vis de la glène G est ainsi ajustable par le chirurgien.

Egalement à titre de variante non représentée, on peut prévoir que, au sein d'un même dispositif de fixation analogue aux dispositifs 3 ou 13, les différents bras 6 ou 16 sont reliés au corps 4 ou 14 par des types de liaison différents entre eux, choisis parmi ceux décrits plus haut, à savoir parmi une liaison rigide, une liaison par déformation souple de matière et une liaison mécaniquement articulée.

En pratique, le corps 4 ou 14 et les bras 6 ou 16, ainsi que, le cas échéant, la bague 18 et la douille 19, sont réalisés en des matériaux permettant de supporter les contraintes mécaniques décrites plus haut, tout en assurant une liaison pérenne entre eux soit rigide, soit déformable élastiquement, soit articulée comme évoqué précédemment. Ainsi, ils peuvent être réalisés en métal ou en polymère, étant d'ailleurs remarqué que les matériaux constituant respectivement le corps 4 et les bras 6 peuvent présenter des compositions différentes. Lorsqu'un polymère est utilisé, ce dernier est avantageusement biorésorbable. Si un alliage métallique est utilisé, ce dernier peut avantageusement être un alliage à mémoire de forme, ce qui permet, lors de la mise en place du corps 4 ou 14 à l'intérieur de la fosse F, de modifier le profil longitudinal incurvé des bras 6, par effet de mémoire de forme, en vue de renforcer l'ancrage du dispositif 3 ou 13 vis-à-vis de la glène G.

Dans tous les cas, le matériau choisi pour constituer le corps 4 ou 14 et les bras 6 ou 16 est avantageusement poreux, pour favoriser la fixation secondaire par colonisation osseuse. D'ailleurs, cette colonisation osseuse est également favorisée en prévoyant que le corps et les bras présentent une structure massive ajourée, facilitant les échanges biologiques dans la fosse F.

Divers aménagements et variantes aux prothèses d'épaule décrites ci-dessus sont par ailleurs envisageables. A titre d'exemples :
- Plutôt que de coopérer par emboîtement tronconique, l'extrémité 42 du corps 4 ou la douille 19 et la face 22A ou 22A' du composant glénoïdien 2 ou 2' peuvent être liées fixement l'une à l'autre par complémentarité d'autres formes, telles qu'une forme en queue d'aronde. D'ailleurs, en complément ou en remplacement de cette coopération par complémentarité de formes, un élément de solidarisation peut être rapporté en plus, tel qu'une vis.
- La section transversale des bras 6 ou 16 peut présenter un profil extérieur autre que de forme carrée, à condition que, du côté opposé au corps 4 ou 14, chaque bras 6 ou 16 présente une surface d'appui sur la face interne V₄ de la paroi V₃ de la voûte V, fonctionnellement analogue aux surfaces d'appui 62 ou 162 décrites plus haut. Par ailleurs, à titre optionnel, chacune de ces surfaces d'appui présente, notamment suivant la direction longitudinale du bras, un profil en dents et en creux afin d'améliorer l'accroche de cette surface à la paroi V₃ de la voûte V.
- Le nombre de bras 6 ou 16 n'est pas limité à quatre ou trois, étant entendu que, pour des raisons de stabilité, au moins trois de ces bras sont indispensables. Ainsi, cinq ou plus de bras sont possibles.
- La vis de fixation intraosseuse 5 peut être remplacée par tout moyen de fixation analogue, à même de coopérer avec l'extrémité 41 ou 141 du corps 4 ou 14.

## Revendications

1. Dispositif (3 ; 13) de fixation à la glène (G) d'un composant articulaire glénoïdien (2 ; 2') pour prothèse d'épaule (1 ; 1'), comportant un corps (4 ; 14) de support du composant glénoïdien (2 ; 2'), qui est adapté pour s'étendre à l'intérieur de la voûte osseuse corticale (V) de la glène (G), dans lequel
le corps (4 ; 14) est solidairement pourvu d'au moins trois bras (6 ; 16) d'appui sur la face interne (V₄) de la paroi périphérique (V₃) de la voûte (V), **caractérisé en ce que** ces bras d'appui sont adaptés, lorsqu'ils sont appuyés sur la face interne de la paroi périphérique de la voûte, pour délimiter entre eux, autour du corps, des passages libres (63) qui courent sur la face extérieure (4A) du corps.

2. Dispositif suivant la revendication 1, **caractérisé en ce que** le corps (4 ; 14) présente un axe central (X-X) selon lequel le corps s'étend à l'intérieur de la voûte (V), et **en ce que** les bras d'appui (6 ; 16) s'étendent en saillie transversale depuis le corps et sont répartis autour du corps suivant une direction périphérique à son axe.

3. Dispositif suivant l'une des revendications 1 ou 2, **caractérisé en ce que** chaque bras d'appui (6 ; 16) présente, sur son côté opposé au corps (4 ; 14), une surface globalement convexe (62 ; 162) de contact pressant avec la face interne (V₄) de la paroi périphérique (V₃) de la voûte (V).

4. Dispositif suivant l'une des revendications précédentes, **caractérisé en ce qu'**au moins l'un des bras d'appui (6 ; 16) est relié rigidement au corps (4 ; 14).

5. Dispositif suivant l'une quelconque des revendications précédentes, **caractérisé en ce qu'**au moins l'un des bras d'appui (6 ; 16) est relié au corps (4 ; 14) de manière souple, par déformation élastique de matière.

6. Dispositif suivant l'une quelconque des revendications précédentes, **caractérisé en ce qu'**au moins l'un des bras d'appui est relié au corps de manière mécaniquement articulée, notamment autour d'un axe orthoradial à l'axe (X-X) du corps.

7. Dispositif suivant l'une quelconque des revendications précédentes, **caractérisé en ce que** le corps (4) et au moins l'un des bras d'appui (6) sont monobloc.

8. Dispositif suivant l'une quelconque des revendications précédentes, **caractérisé en ce qu'**au moins deux des bras d'appui (16) sont portés par une même pièce (18) adaptée pour être solidarisée au corps (14), en étant notamment reçue à l'intérieur de ce corps qui délimite alors des fentes traversantes (145) de réception desdits au moins deux bras d'appui.

9. Dispositif suivant l'une quelconque des revendications précédentes, **caractérisé en ce que** le corps (4 ; 14) est dimensionné pour être reçu à l'intérieur de la voûte (V), en ménageant autour de lui un espace libre (E) entre le corps et la paroi périphérique (V₃) de cette voûte, lequel espace libre est éventuellement comblé par un greffon osseux spongieux (7) rapporté autour du corps (4 ; 14), entre les bras d'appui (6 ; 16), et **en ce que** le corps est muni, à son extrémité (41 ; 141) tournée vers l'intérieur de la glène, d'un moyen (5) de fixation intraosseuse dans le fond (V₂) de la voûte, tandis que, à son extrémité opposée (42 ; 142), le corps est adapté pour être relié fixement au composant articulaire glénoïdien (2 ; 2').

10. Prothèse d'épaule (1 ; 1'), comportant un dispositif de fixation (3 ; 13), conforme à l'une quelconque des revendications précédentes, et un composant articulaire glénoïdien (2 ; 2'), porté par le corps (4 ; 14) du dispositif de fixation et présentant, à l'opposé de ce corps, une face articulaire (22A ; 22A') indifféremment concave ou convexe.

## Claims

1. Device (3; 13) for fixing to the glene a glenoid articular component (2; 2') for a shoulder prosthesis (1; 1'), including a body (4, 14) for support of the glenoid component (2; 2'), which is suitable to extend within the cortical bony vault (V) of the glene (G), in which the body (4; 14) is integrally provided with at least three arms (6; 16) for bearing against the internal face (V₄) of the peripheral wall (V₃) of the vault (V), **characterised in that** these bearing arms are suitable, when they are applied onto the internal face of the peripheral wall of the vault, to delimit between themselves, around the body, free passages (63) which run across the external face (4A) of the body.

2. Device according to Claim 1, **characterised in that** the body (4; 14) exhibits a central axis (X-X), along which the body extends within the vault (V), and **in that** the bearing arms (6; 16) extend in transverse projection from the body and are distributed around the body following a direction peripheral to its axis.

3. Device according to one of Claims 1 or 2, **characterised in that** each bearing arm (6; 16) exhibits, on its side opposite the body (4; 14), a contact surface (62; 162) which is convex overall, pressing against the internal face (V₄) of the peripheral wall (V₃) of the vault (V).

4. Device according to one of the preceding claims, **characterised in that** at least one of the bearing arms (6; 16) is rigidly connected to the body (4; 14).

5. Device according to any one of the preceding claims, **characterised in that** at least one of the bearing arms (6; 16) is connected to the body (4; 14) in flexible manner by elastic deformation of material.

6. Device according to any one of the preceding claims, **characterised in that** at least one of the bearing arms is connected to the body in mechanically articulated manner, in particular about an axis that is orthoradial to the axis (X-X) of the body.

7. Device according to any one of the preceding claims, **characterised in that** the body (4) and at least one of the bearing arms (6) are cast in one piece.

8. Device according to any one of the preceding claims, **characterised in that** at least two of the bearing arms (16) are carried by the same part (18) which is suitable to be firmly attached to the body (14) by being, in particular, received within this body which then delimits through-slots (145) for receiving said at least two bearing arms.

9. Device according to any one of the preceding claims, **characterised in that** the body (4; 14) is dimensioned to be received within the vault (V), by providing around said body a free space (E) between the body and the peripheral wall (V₃) of this vault, which free space is possibly filled up by a spongy bone graft (7) added around the body (4; 14) between the bearing arms (6; 16), and **in that** the body is provided, at its end (41; 141) facing towards the inside of the glene, with a means (5) for intra-osseous fixing in the bottom (V₂) of the vault, whereas at its opposite end (42; 142) the body is suitable to be connected in fixed manner to the glenoid articular component (2; 2').

10. Shoulder prosthesis (1; 1') including a fixing device (3; 13) according to any one of the preceding claims and a glenoid articular component (2; 2') carried by the body (4; 14) of the fixing device and exhibiting, opposite this body, an articular face (22A; 22A') which is either concave or convex.

## Patentansprüche

1. Vorrichtung (3; 13) zum Befestigen einer Glenoidgelenkkomponente (2; 2') an der Gelenkpfanne (G) für eine Schulterprothese (1; 1'), umfassend einen Körper (4; 14) zur Abstützung der Glenoidkomponente (2; 2'), der ausgebildet ist, sich in das Innere des Corticalknochengewölbes (V) der Gelenkpfanne (G) zu erstrecken, bei der der Körper (4; 14) fest mit mindestens drei Armen (6; 16) zur Abstützung an der Innenfläche (V₄) der Umfangswand (V₃) des Gewölbes (V) versehen ist,
***dadurch gekennzeichnet, dass***
diese Abstützarme angepasst sind, zwischen sich um den Körper herum die freien Durchgänge (63), die auf der Außenfläche (4a) des Körpers verlaufen, zu begrenzen, wenn sie an die Innenfläche der Umfangswand des Gewölbes gedrückt werden.

2. Vorrichtung nach Anspruch 1,
***dadurch gekennzeichnet, dass***
der Körper (4; 14) eine Mittelachse (X-X) aufweist, gemäß der der Körper sich ins Innere des Gewölbes (V) erstreckt, und dass die Abstützarme (6; 16) sich als Quervorsprünge von dem Körper aus erstrecken und um den Körper herum gemäß einer Umfangsrichtung zu seiner Achse verteilt sind.

3. Vorrichtung nach einem der Ansprüche 1 oder 2,
***dadurch gekennzeichnet, dass***
jeder Abstützarm (6; 16) an seiner zum Körper (4; 14) entgegengesetzten Seite eine im Wesentlichen konvexe Fläche (62; 162) für den pressenden Kontakt mit der Innenfläche (V₄) der Umfangswand (V₃) des Gewölbes (V) aufweist.

4. Vorrichtung nach einem der vorhergehenden Ansprüche,
***dadurch gekennzeichnet, dass***
mindestens einer der Abstützarme (6; 16) starr mit dem Körper (4; 14) verbunden ist.

5. Vorrichtung nach einem beliebigen der vorhergehenden Ansprüche,
***dadurch gekennzeichnet, dass***
mindestens einer der Abstützarme (6; 16) in durch elastische Materialverformung nachgiebiger Weise mit dem Körper (4; 14) verbunden ist.

6. Vorrichtung nach einem beliebigen der vorhergehenden Ansprüche,
***dadurch gekennzeichnet, dass***
mindestens einer der Abstützarme mit dem Körper in mechanisch gelenkiger Weise, insbesondere um eine Achse ortho-radial zur Achse (X-X) des Körpers verbunden ist.

7. Vorrichtung nach einem beliebigen der vorhergehenden Ansprüche,
***dadurch gekennzeichnet, dass***
der Körper (4) und mindestens einer der Abstützarme (6) aus einem Stück bestehen.

8. Vorrichtung nach einem beliebigen der vorhergehenden Ansprüche,
***dadurch gekennzeichnet, dass***
mindestens zwei der Abstützarme (16) von einem selben Teil (18) getragen werden, das angepasst ist, mit dem Körper (14) verbunden zu werden, indem es insbesondere im Inneren dieses Körpers aufgenommen ist, der dann Durchgreifspalte (145) zur Aufnahme der mindestens zwei Abstützarme begrenzt.

9. Vorrichtung nach einem beliebigen der vorhergehenden Ansprüche,
***dadurch gekennzeichnet, dass***
der Körper (4; 14) dimensioniert ist, um im Inneren des Gewölbes (V) aufgenommen zu werden, indem um ihn ein freier Raum (E) zwischen dem Körper und der Umfangswand (V₃) dieses Gewölbes eingearbeitet ist, wobei dieser freie Raum gegebenenfalls mit einem schwammförmigen Knochentransplantat (7) aufgefüllt ist, das um den Körper (4; 14) zwischen den Abstützarmen (6; 16) eingebracht ist, und dass der Körper an seinem in das Innere der Gelenkpfanne gerichteten Ende (41; 141) mit einem Mittel (5) zur Befestigung in dem Knochen in dem Boden (V₂) des Gewölbes versehen ist, während der Körper an seinem entgegengesetzten Ende (42; 142) angepasst ist, um fest mit der Glenoidgelenkkomponente (2; 2') verbunden zu werden.

10. Schulterprothese (1; 1'), eine Befestigungsvorrichtung (3; 13) nach einem beliebigen der vorhergehenden Ansprüche und eine Glenoidgelenkkomponente (2;2') umfassend, die von dem Körper (4; 14) der Befestigungsvorrichtung getragen wird und entgegengesetzt zu diesem Körper eine Gelenkfläche (22A; 22A'), ob konkav oder konvex, aufweist.
